# EUROPÄISCHE PATENTSCHRIFT

(11) **EP 3 066 997 B1**
(45) Veröffentlichungstag und Bekanntmachung des Hinweises auf die Patenterteilung: **20.10.2021**
(21) Anmeldenummer: 16156794.6
(22) Anmeldetag: 22.02.2016
(51) Int. Cl.: A61B 17/221, A61B 17/3205

(54) **ENDOSKOPISCHE VORRICHTUNG**
ENDOSCOPIC DEVICE
DISPOSITIF ENDOSCOPIQUE

(30) Priorität: 11.03.2015 DE 102015103602
(43) Veröffentlichungstag der Anmeldung: 14.09.2016
(73) Patentinhaber: FUJIFILM MEDWORK GMBH, 91315 Höchstadt/Aisch (DE)
(72) Erfinder: Stirnweiß, Matthias, 91475 Lonnerstadt (DE)
(74) Vertreter: Carstensen, Lars

(56) Entgegenhaltungen:
- DE-A1- 3 316 260
- DE-A1- 3 717 657
- DE-A1- 19 924 639
- DE-U1-202009 015 860
- US-A- 3 955 578
- US-A- 5 098 441

## Beschreibung

Die Erfindung betrifft eine endoskopische Vorrichtung mit einem an einem distalen Ende eines Tubus' angeordneten Instrument, das über ein innerhalb eines Lumens verlaufendes Seil und eine proximal an diesem angreifende Betätigungseinrichtung betätigbar ist, wobei Betätigungselemente der Betätigungseinrichtung zueinander längsverschiebbar geführt sind, von denen ein Betätigungselement zumindest abschnittsweise mit einem Außengewinde versehen ist, in das ein Innengewinde einer Stellmutter eingreift, und wobei eine Stirnfläche der Stellmutter derart mit einer dieser zugewandten Anlagefläche des anderen Betätigungselements zusammenwirkt, dass das Seil über die Stellmutter spannbar ist.

Derartige endoskopische Vorrichtungen werden über einen Arbeitskanal eines Endoskops in den Gastrointestinaltrakt des Patienten eingeführt, wobei das im distalen Endbereich des Tubus' vorgesehene Instrument als Polypektomieschlinge, Steinfangkorb, Schneideinrichtung, Greifer usw. ausgebildet sein kann. Ein Steinfangkorb wird verwendet, um Steine aus einem Gallen- und Pankreasgang, einer Niere, einer Harnblase oder einem Harnleiter auf natürlichem Wege, also ohne einen chirurgischen Eingriff zu entfernen. Der am distalen Ende der Vorrichtung angeordnete Steinfangkorb, auch Dormiakörbchen genannt, wird zumeist über einen in einem separaten Lumen geführten Führungsdraht bis zu dem im Gallenkanal oder in einem anderen Organ befindlichen Stein geführt. Dann öffnet sich der über das Seil aus dem Tubus herausgeschobene Steinfangkorb aufgrund seiner elastischen Vorspannung und erreicht seine größte radiale Erstreckung.

Nun wird der Steinfangkorb an den Stein herangeführt, bis dieser in dessen Inneres, also zwischen die Korbdrähte gelangt. Bei einer anschließenden Zugbewegung des Seils relativ zum Tubus zieht sich der Steinfangkorb fest um den Stein zusammen und umschließt diesen, so dass er über das Seil sowie den Tubus, die entsprechenden Körperöffnungen passierend, sicher entfernt werden kann. Erhebliche Probleme treten dann auf, wenn der Stein derartige Abmessungen besitzt, dass er nicht durch Körperöffnungen, wie z.B. den Gallenkanal oder den Harnleiter gezogen werden kann. Eine gewaltsame Entfernung des Steins würde zu beträchtlichen Verletzungen des zu behandelnden Patienten führen.

Neben der Einrichtung zum Einfangen des Steines und zu dessen Entfernung hat es daher im Stand der Technik eine Vielzahl von Lösungen gegeben, wie der bezüglich seiner Abmessungen zu große Stein zertrümmert werden kann, damit er durch die entsprechenden Körperöffnungen passt. Die zu diesem Zweck eingesetzten Lithotriptoren lassen sich im Wesentlichen in solche unterscheiden, die mittels Ultraschall oder mechanisch den im Körper des Patienten befindlichen Stein zertrümmern.

Mittels einer Polypektomieschlinge werden Polypen im Magen oder im Darmtrakt abgetrennt. Die vorgenannten Schneideinrichtungen können beispielsweise als Papillotom ausgebildet sein, mit dessen außerhalb des Tubus verlaufenden Schneiddraht die Papilla Vateri geöffnet wird, um die Gallensteine anschließend mittels eines Steinfangkorbes zu entfernen. Mit einem als Greifer ausgeführten Instrument können Fremdkörper aus dem Magen entfernt werden.

Am proximalen Ende des Tubus befindet sich eine Betätigungseinrichtung, die im wesentlichen aus einem mit dem Tubus verbundenen Führungsschaft und einem auf diesem manuell längsverschiebbaren Stellelement besteht. Dabei greift das Stellelement zumeist über eine Zugstange an einem Seil an, welches distal derart mit dem jeweiligen Instrument verbunden ist, dass es auf dieses eine Betätigungskraft übertragen kann.

Eine Vorrichtung der im Oberbegriff des Patentanspruchs 1 angegebenen Gattung ist aus der US 5,098,441 A bekannt. Diese zeigt einen endoskopischen Lithotriptor mit einem Fangkorb, der mit einem distalen Ende eines Zugseils verbunden ist. Das Zugseil verläuft innerhalb eines Tubus', wobei der Tubus und das Zugseil proximal jeweils mit zueinander längsverschieblichen Betätigungselementen einer Betätigungseinrichtung verbunden sind. Dabei ist das am Tubus angreifende Betätigungselement als hohlzylindrischer Handgriff ausgebildet, der sich über eine kegelstumpfförmige Nabe am proximalen Ende des Tubus' abstützt. Das weitere Betätigungselement ist als mit einem Außengewinde versehene Zugstange ausgeführt, wobei das Zugseil durch diese Zugstange hindurch verläuft und in dieser über eine an dessen proximalem Ende vorgesehene Klemmeinrichtung fixiert wird. Diese Klemmeinrichtung soll nach der Art eines Bohrfutters, also mit Klemmbacken ausgebildet sein. Zum Spannen des Fangkorbes mit einer auf das Zugseil ausgeübten Zugkraft, die ausreicht, um einen vom Fangkorb aufgenommenen Stein zu zertrümmern, ist auf der mit dem Zugseil verbunden Zugstange eine Stellmutter angeordnet, deren eine Stirnseite mit einer Anlagefläche des hohlzylindrischen Handgriffs zusammenwirkt.

Weiterhin geht aus der DE 199 24 639 A1 ein Bedienteil eines endoskopischen Behandlungsinstruments hervor. Das Behandlungsgerät weist ein distal mit einem Fangkorb verbundenes Zugseil auf, das axial beweglich in einem Tubus geführt ist. Dabei nimmt der Tubus an seinem proximalen Ende ein zylindrisches Element des Bedienteils auf, während ein um 180° umgebogenes Ende des Zugseils zwischen im Inneren eines ringförmigen Griffstücks vorgesehenen Drahtbefestigungselementen eingeklemmt ist und das Griffstück verschiebbar auf dem zylindrischen Element geführt ist. Weiterhin soll ein Hub des Zugseils gegenüber dem Tubus in Öffnungsrichtung des Fangkorbs durch einen verstellbaren Anschlag begrenzt werden. Dieser Anschlag besteht aus einem auf der Außenmantelfläche des zylindrischen Elements geführten Ring, der auf diesem in unterschiedlichen Stellungen fixierbar ist.

Außerdem zeigt die US 3,955,578 A eine endoskopische Vorrichtung, die an ihrem distalen Ende eine Polypektomieschlinge aufweist. Die proximal mit dem Tubus und dem Seil verbundene Betätigungseinrichtung ist mit einem gegenüber einem Führungsschaft verschiebbaren Stellelement versehen, das zwei Fingerringe für eine Längsbewegung des Zugseils aufweist. Außerdem ist am Stellelement ein Drehgriff vorgesehen, über den eine rotative Bewegung auf den Tubus und das Zugseil, also somit auf die Polypektomieschlinge übertragen werden kann.

Die DE 32 16 178 A1 offenbart eine Vorrichtung zur mechanischen Lithotripsie und Entfernung von Steinen, die sich im Gallengang gebildet haben. Diese Vorrichtung besteht aus einem Extraktor und einer mit diesem kombinierbaren Einrichtung zur Lithotripsie. Dabei weist der Extraktor eine Betätigungseinrichtung in Form eines mit einer Schiebestange verbundenen Handgriffs und eine die Schiebestange aufnehmende Hülse auf. Die Schiebestange ist teleskopisch in der Hülse geführt und mit ihrem vom Handgriff abgewandten Ende an dem zum Steinfangkorb führenden Zugseil fixiert. Wird folglich die Schubstange axial in der Hülse verlagert, so führt das, je nach Bewegungsrichtung, zu einem Öffnen oder einem Schließen des Steinfangkorbes, so dass ein beispielsweise im Gallengang vorhandener Stein von dem Steinfangkorb aufgenommen und durch eine Bewegung der gesamten Vorrichtung in proximaler Richtung aus dem Körper des Patienten entfernt werden kann.

Zusätzlich zu diesem Extraktor ist gemäß der DE 32 16 178 A1 ein separater Lithotriptor vorgesehen, der bei Bedarf an dem Stellelement fixiert wird. Es handelt sich dabei um ein Spannelement mit radial zu diesem verlaufenden Halterungen, die nach der Anbringung des Lithotriptors einerseits am Handgriff des ersten Stellelements und andererseits an einem mit der Hülse verbundenen Kopfstück angreifen. Wird festgestellt, dass der Stein Abmessungen besitzt, die dessen problemlose Entfernung verhindern, so wird über den an der Betätigungseinrichtung angesetzten Lithotriptor eine derart starke Zugkraft auf das Seil ausgeübt, dass der im Steinfangkorb befindliche Stein in mehrere kleine Partikel zerfällt. Diese Partikel lassen sich dann mit dem Steinfangkorb entfernen.

Aufgabe der vorliegenden Erfindung ist es, eine Vorrichtung der im Oberbegriff des Anspruch 1 angegebenen Gattung zu schaffen, die Verbesserungen sowohl hinsichtlich der Handhabung als auch der Herstellkosten aufweist, wobei mittels der Betätigungseinrichtung der Vorrichtung neben präzisen und mit hohen Verstellgeschwindigkeiten durchgeführten Stellbewegungen eine erhöhte Zugkraft auf das Seil übertragen werden und/oder das Stilelement in einer vorgegebenen Stellung fixiert werden.

Diese Aufgabe wird, ausgehend vom Oberbegriff des Patentanspruchs 1 in Verbindung mit dessen kennzeichnenden Merkmalen gelöst. Die vom Patentanspruch 1 abhängigen Patentansprüche beinhalten erfindungsgemäße Weiterbildungen dieser Lösung.

Danach soll das zumindest abschnittsweise mit dem Außengewinde versehene Betätigungselement als Führungsschaft ausgebildet sein, der zumindest mittelbar mit dem Tubus verbunden ist und an seinem proximalen Ende einen Daumenring aufnimmt, und dass das andere Betätigungselement als ein an diesem, zur Einleitung der Betätigungsbewegung in Richtung eines proximalen Endes des Führungsschafts bewegbares, mit der Anlagefläche versehenes Stellelement ausgebildet ist, an welchem zwei Fingerringe vorgesehen sind, und dass das Stellelement in seiner gegenüber dem Führungsschaft eingenommenen Position fixierbar ist.

Somit kann zunächst durch das Verschieben des Stellelements gegenüber dem Führungsschaft eine Verstellung des Instruments vorgenommen werden, wobei bei Erreichen einer optimalen Einstellung des Instruments eventuell an der Betätigungseinrichtung ein haptisches Signal spürbar ist. Wenn es sich bei dem Instrument um einen Steinfangkorb handelt, ist während dessen Verstellung an der Betätigungseinrichtung spürbar, wenn der Stein von diesem erfasst und umschlossen ist. Bei einer Schneideinrichtung, wie z.B. einem Papillotom können unterschiedliche Schneiddrahtlängen und somit unterschiedliche Krümmungen des distalen Endes des Tubus' signalisiert werden.

Anschließend kann die distal des Stellelements vorgesehene Stellmutter soweit auf dem Außengewinde des Führungsschafts verdreht werden, bis sie mit ihrer entsprechenden Stirnfläche am Stellelement anliegt. Dadurch ist das Instrument in der entsprechenden Stellung fixiert bzw. positioniert. Bei einem Steinfangkorb bedeutet das, dass dieser sich nicht mehr öffnen kann, was anderenfalls zur Folge hätte, dass der Stein wieder freigegeben würde.

Somit lässt sich für eine endoskopische Vorrichtung der genannten Gattung eine Betätigungseinrichtung schaffen, die aus einer geringen Anzahl von Einzelteilen herstellbar ist und einen einfachen Aufbau aufweist, so dass sie für eine Fertigung in einer Großserie geeignet ist. Die Vorrichtung ist intuitiv bedienbar. Bei der Verstellung des Instruments können sehr präzise und eine hohe Stellgeschwindigkeit aufweisende Stellbewegungen ausgeführt werden, da das durch das erläuterte Verschieben des Stellelements gegenüber dem Führungsschaft geschieht. Anschließend wird diese Einstellung des Instruments festgelegt, oder die Stellbewegung wird mit Hilfe der mit dem Führungsschaft zusammenwirkenden Stellmutter mit einer erhöhten Zugkraft am Seil fortgesetzt. Soll eine präzise Einstellung der Vorrichtung vorgenommen werden, die durch ein manuelles Verschieben des Stellelements gegenüber dem Führungsschaft nicht erzielbar ist, so kann das auch direkt mit Hilfe der Stellmutter geschehen. An der Betätigungseinrichtung sind die jeweiligen Stellungen des Instruments ablesbar, also beispielsweise die freie Schneiddrahtlänge beim Papillotom, der Öffnungsgrad des Steinfangkorbes oder der Polypektomieschlinge usw.

Bei einer Verwendung der endoskopischen Vorrichtung in Verbindung mit einem Steinfangkorb kann diese außerdem als mechanischer Lithotriptor verwendet werden. Zu diesem Zweck wird zunächst, wie bereits erläutert, die Stellmutter soweit in Richtung des Stellelements geschraubt, bis sie an diesem anliegt. Zur axialen Abstützung des Steinfangkorbes wird anschließend auf dem Tubus ein Spiralschlauch bis an den Steinfangkorb verschoben. Nach der Platzierung dieses Widerlagers kann dann wahlweise über eine manuelle Verschiebung des Stellelements gegenüber dem Führungsschaft oder durch Erzeugen einer deutlich höheren Zugkraft mittels der Stellmutter der Steinfangkorb derart gespannt werden, dass der Stein zertrümmert wird.

Außerdem sollen am proximalen Ende des Führungsschafts ein Daumenring und am Stellelement zwei Fingerringe vorgesehen sein. Das Stellelement, das in einem Führungsschlitz des Führungsschafts geführt ist, nimmt eine Zugstange auf, die an ihrem distalen Ende mit dem Seil verbunden ist. Dabei kann der Führungsschaft als Gewindespindel ausgebildet sein, an dem der Daumenring formschlüssig oder durch Kleben befestigt ist. Im Bereich der Führungsschlitze weist die Gewindespindel Abflachungen auf, die parallel zueinander verlaufen und an denen das Stellelement geführt ist. Das Gewinde der Gewindespindel ist vorzugsweise als Flach-, Trapez- oder Rundgewinde ausgebildet. Dabei kann das Gewinde beispielsweise bei einem Durchmesser vom 24 mm eine Gewindesteigung von 5 mm aufweisen. Denkbar ist aber auch, ein Feingewinde mit einer sehr kleinen Gewindesteigung vorzusehen. Durch die Verwendung eines Feingewinde lassen sich mit einem geringen an der Stellmutter aufgebrachten Drehmoment am Seil hohe Zugkräfte erzielen.

Demgegenüber ist beim gattungsbildenden Stand der Technik nach der Druckschrift US 5,098,441 A das mit dem Außengewinde versehene Betätigungselement als Zugstange ausgebildet, die mit einem proximalen Ende eines Zugseils verbunden ist. Dabei verläuft das Zugseil durch das Innere dieser Zugstange und ist in dieser über eine an deren proximalem Ende vorgesehene Klemmeinrichtung fixiert. Ebenfalls im Gegensatz zur erfindungsgemäßen Lehre ist der Tubus an einem hohlzylindrischen Handgriff abgestützt. Eine normale Verstellung des Instruments erfolgt dadurch, dass die Zugstange innerhalb des hohlzylindrischen Handgriffes verschoben wird. Außerdem weist die Betätigungseinrichtung nach dem Stand der Technik keine Fingerringe und keinen Daumenring für deren Verstellung auf. Bei der DE 199 24 639 A1 ist zwar der Tubus am zylindrischen Element des Bedienteils abgestützt und das ringförmigen Griffstück mit dem proximalen Ende des Zugseils verbunden, das zylindrische Element weist aber kein Außengewinde mit einer darauf angeordneten Stellmutter auf. Vielmehr ist ein als Ring ausgebildeter Anschlag vorgesehen, der sich in unterschiedliche Positionen verschieben und in diesen fixieren lässt. Dieser soll die Öffnungsbewegungen des Fangkorbes, also den Hub des Zugseils in Öffnungsrichtung begrenzen.

Vorzugsweise kann die Vorrichtung zum Entfernen von Steinen aus einem Gallengang oder aus einem Harnsystem vorgesehen sein, wobei das Instrument als Steinfangkorb ausgebildet ist. Dieser Steinfangkorb ist über die am proximalen Ende des Seils angreifende Betätigungseinrichtung in eine Öffnungs- oder Schließbewegung verstellbar, wobei der Stein nach seiner Aufnahme im Inneren des Steinfangkorbes in diesem durch die Schließbewegung fixierbar ist, die durch ein manuelles Verschieben des Stellelements gegenüber dem Führungsschaft erzielt wird. Anschließend wird der Stein durch ein Spannen des Seils mittels der Stellmutter in Zugrichtung zertrümmert. Im Zusammenhang mit der Anordnung von Fingerringen am Stellelement ist vorgesehen, dass das Stellelement zweiteilig ausgebildet ist, wobei jede Stellelementhälfte einen Fingerring aufweist und die Stellelementhälften beidseitig des Führungsschafts in einen in diesem vorgesehenen Führungsschlitz eingefügt und aneinander befestigt sind. Dabei können die Stellelementhälften über quer verlaufende Schrauben aneinander befestigt sein. Die Zugstange oder das Seil sind in diesem Fall zwischen den Stellelementhälften eingeklemmt.
Schließlich ist vorgesehen, die Stellmutter an ihrer Außenmantelfläche ballig auszubilden und mit Längsnuten oder einer Rändelung zu versehen. Der Chirurg wird mit einer Hand die Schnellverstellung über die Fingerringe vornehmen und mit der anderen Hand die Stellmutter nachführen. Beim Anspannen der Stellmutter kann er den Führungsschaft mit Hilfe der Fingerringe abstützen.

Die vorliegende Offenbarung ist nicht auf die angegeben Kombination der Merkmale des Patentanspruchs 1 und der abhängigen Patentansprüche beschränkt. Die Bezugnahme der Patentansprüche auf die Zeichnung durch die Verwendung von Bezugszeichen soll den Schutzumfang der Patentansprüche nicht beschränken.

Zur weiteren Erläuterung der Erfindung wird auf die Zeichnung verwiesen, in der ein Ausführungsbeispiel vereinfacht dargestellt ist. Es zeigen:
- Figur 1: ein proximales Ende einer endoskopischen Vorrichtung mit einer erfindungsgemäß ausgebildeten Betätigungseinrichtung,
- Figur 2: ein distales Ende der Vorrichtung mit einem als Steinfangkorb ausgebildeten Instrument, wobei dieser sich in seiner geöffneten Stellung befindet,
- Figur 3: eine perspektivische Ansicht der Betätigungseinrichtung der Figur 1,
- Figur 4: einen Längsschnitt durch die in Figur 3 dargestellte Betätigungseinrichtung und
- Figur 5: einen gegenüber der Darstellung nach der Figur 4 um 90° versetzten Längsschnitt durch die in Figur 3 dargestellte Betätigungseinrichtung.

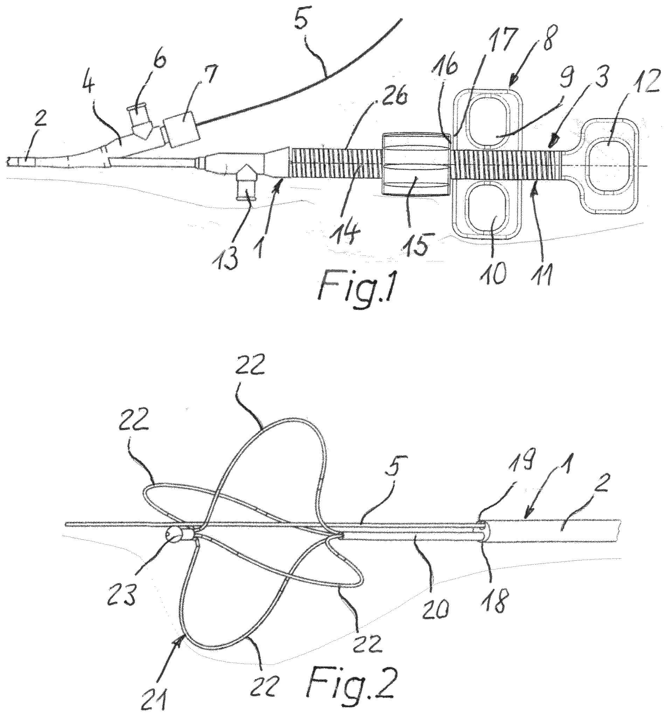

In der Figur 1 ist mit 1 eine endoskopische Vorrichtung bezeichnet, die aus einem Tubus 2 und einer Betätigungseinrichtung 3 besteht. Dabei ist zwischen der Betätigungseinrichtung 3 und dem Tubus 2 eine Schleuse 4 angeordnet, über welche in den Tubus 2 ein Führungsdraht 5 eingeführt werden kann. Diese Schleuse 4 ist weiterhin mit einem Stutzen 6 zur Zuführung von Spülflüssigkeiten versehen, wobei die Schleuse 4 durch eine den Führungsdraht 5 umgebende Kappe 7 abgedichtet ist. Wie der Figur 1 weiterhin entnommen werden kann, verläuft die Schleuse 4 unter einem spitzen Winkel zum Tubus 2.

Die Betätigungseinrichtung 3 weist ein Stellelement 8 auf, das mit zwei Fingerringen 9 und 10 versehen ist, und einen konzentrisch zum Tubus 2 verlaufenden Führungsschaft 11, der an seinem proximalen Ende einen Daumenring 12 aufnimmt, auf. Dabei ist das Stellelement 8, wie noch näher erläutert werden wird, längsverschiebbar am Führungsschaft 11 geführt. Zwischen dem Tubus 2 und der Betätigungseinrichtung 3 ist eine weitere Schleuse 13 angeordnet, über die ebenfalls eine Flüssigkeit zugeführt werden kann.

Der Führungsschaft 11 ist mit einem Außengewinde 14 versehen, das sich bei dem dargestellten Ausführungsbeispiel proximal bis zum Daumenring 12 erstreckt und auf das eine Stellmutter 15 aufgeschraubt ist. Dabei befindet sich die Stellmutter 15 zwischen dem distalen Ende des Führungsschafts 11 und dem Stellelement 8 und wirkt mit ihrer dem Stellelement 8 zugewandten Stirnfläche 16 mit einer Anlagefläche 17 des Stellelements 8 zusammen. Dadurch lässt sich mittels der Stellmutter 15 die Position des Stellelements 8 fixieren, nachdem zuvor durch Verschiebung das Stellelement 8 über den Daumenring 12 und die Fingerringe 9 und 10 in eine entsprechende Position gebracht wurde. Außerdem kann anschließend kann mit der Stellmutter 15 eine erhöhte Kraft am Stellelement 8 aufgebracht werden, die sich, wie nachfolgend im Zusammenhang mit der Figur 2 erläutert, auswirkt.

Wie aus der Figur 2, die den distalen Endbereich der endoskopischen Vorrichtung zeigt, hervorgeht, sind in dem Tubus zwei Lumen 18 und 19 vorgesehen, wobei das eine Lumen 18 ein Seil 20 aufnimmt, das proximal zumindest mittelbar mit dem Stellelement 8 verbunden ist. Innerhalb des anderen Lumens 19 verläuft der zuvor beschriebene Führungsdraht 5. Der in der Figur 1 dargestellte Führungsschaft 11 ist fest mit dem Tubus 2 verbunden, so dass sich aufgrund einer Relativbewegung des Stellelements 8 gegenüber dem Führungsschaft 11 das Seil 20 innerhalb des Tubus 2 hin und her bewegen lässt.

An seinem distalen Ende nimmt das Seil 20 einen Steinfangkorb 21 auf, dessen Korbdrähte 22 in der Darstellung einen derartigen Verlauf aufweisen, dass sich der Steinfangkorb 21 in seiner geöffneten Stellung befindet, in welcher er einen Stein aufnehmen kann. Um diese Einstellung des Steinfangkorbes 21 vorzunehmen, ist das Stellelement 8 der in Figur 1 dargestellten Betätigungseinrichtung 3 gegenüber dem Führungsschaft 11 in eine distale Position verschoben. Die Korbdrähte 22 sind an ihren jeweiligen Enden gebündelt, und zwar einerseits am Ende des Seils 20 und andererseits in einem patronenförmigen Führungselement 23. Wird das Stellelement 8 in Richtung einer proximalen Position verschoben, so verengt sich der Steinfangkorb 21, was zunächst zum Fixieren des aufgenommenen Steins erforderlich ist. Wird der den Stein aufnehmende Steinfangkorb 21 weiter verengt, so wirkt er als mechanischer Lithotriptor, d.h., der Stein wird zerquetscht, wobei die Konkremente des Steins anschließend mit dem Steinfangkorb 21 entfernt werden können.

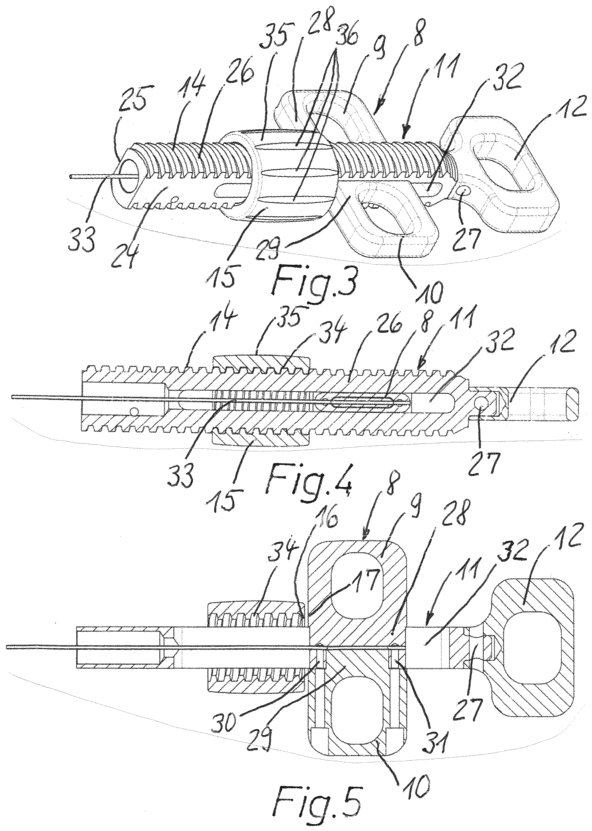

Weitere Details der Betätigungseinrichtung können den Figuren 3 bis 5 entnommen werden. Aus diesen geht hervor, dass der Führungsschaft 11 eine mit Abflachungen 24 und 25 versehene Gewindespindel 26 aufweist, an deren proximalem Ende der Daumenring 12 über einen Passstift 27 befestigt ist. Das mit den Fingerringen 9 und 10 versehene Stellelement 8 ist zweiteilig aus zwei Stellelementhälften 28 und 29 hergestellt, wobei diese über Schrauben 30 und 31 aneinander befestigt sind. Dabei sind die miteinander verspannten Stellelementhälften 28 und 29 jeweils an einer der Abflachungen 24 bzw. 25 geführt und greifen in einen als Langloch ausgebildeten Führungsschlitz 32 der Gewindespindel 26 ein. Diese Abschnitte der beiden Stellelementhälften 28 und 29 nehmen an ihren Berührungsflächen gemeinsam eine Zugstange 33 auf, die an ihrem distalen Ende mit dem Seil 20 verbunden ist.

Die Stellmutter 15 ist mit einem Innengewinde 34 versehen, über das sie auf das Außengewinde 14 der Gewindespindel 26 aufgeschraubt ist. Die beiden Gewinde 14 und 34 sind als Trapezgewinde ausgeführt. Eine Außenmantelfläche 35 der Stellmutter 15 ist ballig ausgebildet und weist mehrere Längsnuten 36 auf.

### Bezugszeichenliste

- 1: endoskopische Vorrichtung
- 2: Tubus
- 3: Betätigungseinrichtung
- 4: Schleuse
- 5: Führungsdraht
- 6: Stutzen
- 7: Kappe
- 8: Stellelement
- 9: Fingerring
- 10: Fingerring
- 11: Führungsschaft von 3
- 12: Daumenring
- 13: Schleuse
- 14: Außengewinde
- 15: Stellmutter
- 16: Stirnfläche von 15
- 17: Anlagefläche von 8
- 18: Lumen
- 19: Lumen
- 20: Seil
- 21: Steinfangkorb
- 22: Korbdrähte
- 23: Führungselement
- 24: Abflachung
- 25: Abflachung
- 26: Gewindespindel
- 27: Passstift
- 28: Stellelementhälfte
- 29: Stellelementhälfte
- 30: Schraube
- 31: Schraube
- 32: Führungsschlitz
- 33: Zugstange
- 34: Innengewinde von 15
- 35: Außenmantelfläche von 15
- 36: Längsnuten von 15

## Patentansprüche

1. Endoskopische Vorrichtung (1) mit einem an einem distalen Ende eines Tubus' (2) angeordneten Instrument, das über ein innerhalb eines Lumens (18) verlaufendes Seil (20) und eine proximal an diesem angreifende Betätigungseinrichtung (3) betätigbar ist, wobei Betätigungselemente (8 und 11) der Betätigungseinrichtung (3) zueinander längsverschiebbar geführt sind, von denen ein Betätigungselement zumindest abschnittsweise mit einem Außengewinde (14) versehen ist, in das ein Innengewinde (34) einer Stellmutter (15) eingreift, und wobei eine Stirnfläche (16) der Stellmutter (15) derart mit einer dieser zugewandten Anlagefläche (17) des anderen Betätigungselements zusammenwirkt, dass das Seil (20) über die Stellmutter (15) spannbar ist, **dadurch gekennzeichnet, dass** das zumindest abschnittsweise mit dem Außengewinde (14) versehene Betätigungselement als Führungsschaft (11) ausgebildet ist, der zumindest mittelbar mit dem Tubus (2) verbunden ist und an seinem proximalen Ende einen Daumenring (12) aufnimmt, dass das andere Betätigungselement als ein an diesem längsverschiebbar geführtes, zur Einleitung der Betätigungsbewegung in Richtung eines proximalen Endes des Führungsschafts (11) bewegbares mit der Anlagefläche versehenes Stellelement (8) ausgebildet ist, an welchem zwei Fingerringe (9 und 10) vorgesehen sind, und dass das Stellelement (8) in seiner gegenüber dem Führungsschaft (11) eingenommenen Position fixierbar ist.

2. Endoskopische Vorrichtung nach Patentanspruch 1, **dadurch gekennzeichnet, dass** die Vorrichtung zum Entfernen von Steinen aus einem Gallengang oder aus einem Harnsystem vorgesehen und das Instrument als Steinfangkorb (21) ausgebildet ist, der über die am proximalen Ende des Seils (20) angreifende Betätigungseinrichtung (3) in eine Öffnungs- oder Schließbewegung verstellbar ist, wobei der Stein nach seiner Aufnahme im Inneren des Steinfangkorbes (21) in diesem durch die Schließbewegung fixierbar ist, die durch ein manuelles Verschieben des Stellelements (8) gegenüber dem Führungsschaft (11) erzielt wird, und dass der Stein durch ein anschließendes Spannen des Seils (20) in Zugrichtung, das mittels der Stellmutter (15) erfolgt, zertrümmerbar ist.

3. Endoskopische Vorrichtung nach Patentanspruch 1, **dadurch gekennzeichnet, dass** das Stellelement (8) mittels der Stellmutter (15) derart gegenüber dem Führungsschaft (11) vorspannbar ist, dass die über das Seil (20) auf den Steinfangkorb (21) übertragene Schließkraft zur mechanischen Lithotripsie des Steins führt.

4. Endoskopische Vorrichtung nach Patentanspruch 1, **dadurch gekennzeichnet, dass** das Stellelement (8) zweiteilig ausgebildet ist, wobei jede Stellelementhälfte (28 und 29) einen Fingerring (9, 10) aufweist und die Stellelementhälften (28 und 29) beidseitig des Führungsschafts (11) in einen in diesem vorgesehenen Führungsschlitz (32) eingefügt und aneinander befestigt sind.

5. Endoskopische Vorrichtung nach Patentanspruch 4, **dadurch gekennzeichnet, dass** die Stellelementhälften (28 und 29) miteinander verschraubt sind.

6. Endoskopische Vorrichtung nach Patentanspruch 5, **dadurch gekennzeichnet, dass** das proximale Ende des Seils (20) zwischen den miteinander verspannten Stellelementhälften (28 und 29) fixiert ist.

7. Endoskopische Vorrichtung nach Patentanspruch 4, **dadurch gekennzeichnet, dass** der als Gewindespindel (26) ausgebildete Führungsschaft (11) zwei parallel verlaufende Abflachungen (24 und 25) aufweist, von denen der Führungsschlitz (32) ausgeht, wobei die Stellelementhälften (28 und 29) an den ebenen Flächen der Abflachungen (24 und 25) geführt sind.

8. Endoskopische Vorrichtung nach Patentanspruch 1, **dadurch gekennzeichnet, dass** die Stellmutter (15) an ihrer Außenmantelfläche (35) ballig ausgebildet und mit Längsnuten (36) versehen ist.

## Claims

1. Endoscopic device (1) having an instrument which is arranged at a distal end of a tube (2) and which is actuatable via a cable (20) extending within a lumen (18) and via an actuator (3) engaging proximally on said cable (20), wherein actuating elements (8 and 11) of the actuator (3) are guided longitudinally displaceably relative to each other, of which one actuating element is provided at least in part with an outer thread (14) in which an inner thread (34) of an adjusting nut (15) engages, and wherein an end face (16) of the adjusting nut (15) interacts with a contact face (17), directed towards it, of the other actuating element, in such a way that the cable (20) can be tensioned via the adjusting nut (15), **characterized in that** the actuating element provided at least in part with the outer thread (14) is designed as a guide shaft (11) which is connected at least indirectly to the tube (2) and, at its proximal end, receives a thumb ring (12), **in that** the other actuating element is designed as an adjusting element (8) which is provided with the contact face and which is guided longitudinally displaceably on the guide shaft (11) and, in order to initiate the actuating movement, is movable in the direction of a proximal end of the guide shaft (11), on which adjusting element (8) two finger rings (9 and 10) are provided, and **in that** the adjusting element (8) is fixable in its position adopted with respect to the guide shaft (11).

2. Endoscopic device according to Claim 1, **characterized in that** the device is provided for removing stones from a bile duct or from a urinary system, and the instrument is designed as a stone-catching basket (21) which is adjustable in an opening or closing movement via the actuator (3) engaging at the proximal end of the cable (20), wherein the stone, after it has been received in the interior of the stone-catching basket (21), is fixable in the latter by the closing movement, the latter being obtained by a manual displacement of the adjusting element (8) relative to the guide shaft (11), and **in that** the stone can be crushed by a subsequent tensioning of the cable (20) in the pulling direction, which is effected by means of the adjusting nut (15).

3. Endoscopic device according to Claim 1, **characterized in that**, by means of the adjusting nut (15), the adjusting element (8) can be pretensioned relative to the guide shaft (11) in such a way that the closing force transmitted to the stone-catching basket (21) via the cable (20) leads to the mechanical lithotripsy of the stone.

4. Endoscopic device according to Claim 1, **characterized in that** the adjusting element (8) is designed in two parts, wherein each adjusting element half (28 and 29) has a finger ring (9, 10), and the adjusting element halves (28 and 29), on both sides of the guide shaft (11), are inserted into a guide slot (32) provided in the latter and are secured to each other.

5. Endoscopic device according to Claim 4, **characterized in that** the adjusting element halves (28 and 29) are screwed together.

6. Endoscopic device according to Claim 5, **characterized in that** the proximal end of the cable (20) is fixed between the mutually tensioned adjusting element halves (28 and 29).

7. Endoscopic device according to Claim 4, **characterized in that** the guide shaft (11), designed as threaded spindle (26), has two parallel flats (24 and 25), from which the guide slot (32) starts, wherein the adjusting element halves (28 and 29) are guided on the plane surfaces of the flats (24 and 25) .

8. Endoscopic device according to Claim 1, **characterized in that** the outer circumferential surface (35) of the adjusting nut (15) has a convex shape and is provided with longitudinal grooves (36) .

## Revendications

1. Dispositif endoscopique (1) comprenant un instrument, qui est disposé à une extrémité distale d'un tube (2) et qui peut être actionné par le biais d'un câble (20) s'étendant à l'intérieur d'une lumière (18), et un mécanisme d'actionnement (3) qui s'engage avec ledit câble du côté proximal, des éléments d'actionnement (8 et 11) du mécanisme d'actionnement (3) étant guidés l'un par rapport à l'autre de manière coulissante longitudinalement, dont un élément d'actionnement est pourvu au moins par portion d'un filetage extérieur (14) dans lequel s'engrène un filetage intérieur (34) d'un écrou de réglage (15), et une surface frontale (16) de l'écrou de réglage (15) coopérant avec une surface d'appui (17) de l'autre élément d'actionnement dirigé vers ledit écrou de réglage de telle sorte que le câble (20) puisse être tendu par le biais de l'écrou de réglage (15), **caractérisé en ce que** l'élément d'actionnement pourvu au moins par portions du filetage extérieur (14) est conçu comme un arbre de guidage (11), qui est relié au moins indirectement au tube (2) et qui reçoit à son extrémité proximale une bague de pouce (12), **en ce que** l'autre élément d'actionnement est conçu comme un élément de réglage (8) qui est pourvu de la surface d'appui, qui est guidé de manière coulissante longitudinalement sur ledit élément d'actionnement, qui peut être déplacé en direction d'une extrémité proximale de l'arbre de guidage (11) pour amorcer le mouvement d'actionnement et sur lequel deux bagues de doigt (9 et 10) sont prévues, et **en ce que** l'élément de réglage (8) peut être fixé dans sa position occupée par rapport à l'arbre de guidage (11).

2. Dispositif endoscopique selon la revendication 1, **caractérisé en ce que** le dispositif est prévu pour retirer des calculs d'un canal cholédoque ou d'un système urinaire et l'instrument est conçu comme un panier collecteur de calculs (21) qui est déplaçable suivant un mouvement d'ouverture ou de fermeture par le biais du mécanisme d'actionnement (3) agissant sur l'extrémité proximale du câble (20), le calcul pouvant être fixé dans le panier collecteur de calculs (21), après sa réception à l'intérieur de celui-ci, par le mouvement de fermeture qui est obtenu par coulissement manuel de l'élément de réglage (8) par rapport à l'arbre de guidage (11), et **en ce que** le calcul peut être brisé par une contrainte ultérieure du câble (20) dans le sens de traction, laquelle est effectuée au moyen de l'écrou de réglage (15).

3. Dispositif endoscopique selon la revendication 1, **caractérisé en ce que** l'élément de réglage (8) peut être précontraint par rapport à l'arbre de guidage (11) au moyen de l'écrou de réglage (15) de telle sorte que la force de fermeture transmise par le câble (20) au panier collecteur de calculs (21) entraîne la lithotripsie mécanique du calcul.

4. Dispositif endoscopique selon la revendication 1, **caractérisé en ce que** l'élément de réglage (8) est conçu en deux parties, chaque moitié d'élément de réglage (28 et 29) comportant une bague de doigt (9, 10) et les moitiés d'élément de réglage (28 et 29) étant insérées des deux côtés de l'arbre de guidage (11) dans une fente de guidage (32) ménagée dans celui-ci et étant fixées l'une à l'autre.

5. Dispositif endoscopique selon la revendication 4, **caractérisé en ce que** les moitiés d'élément de réglage (28 et 29) sont vissées l'une à l'autre.

6. Dispositif endoscopique selon la revendication 5, **caractérisé en ce que** l'extrémité proximale du câble (20) est fixée entre les moitiés d'élément de réglage (28 et 29) qui sont entretoisées.

7. Dispositif endoscopique selon la revendication 4, **caractérisé en ce que** l'arbre de guidage (11) conçu comme une broche filetée (26) comporte deux méplats parallèles (24 et 25) desquels provient la fente de guidage (32), les moitiés d'élément de réglage (28 et 29) étant guidées sur les surfaces planes des méplats (24 et 25).

8. Dispositif endoscopique selon la revendication 1, **caractérisé en ce que** l'écrou de réglage (15) est convexe sur sa surface latérale extérieure (35) et est pourvu de rainures longitudinales (36).
